(19) Europäisches Patentamt
European Patent Office
Office européen des brevets

(11) Publication number: **0 263 684 A2**

(12) **EUROPEAN PATENT APPLICATION**

(21) Application number: **87308862.9**

(51) Int. Cl.[4]: **A 61 K 7/42**

(22) Date of filing: **06.10.87**

(30) Priority: **06.10.86 US 915531**

(43) Date of publication of application: **13.04.88 Bulletin 88/15**

(84) Designated Contracting States: **AT BE CH DE ES FR GB GR IT LI LU NL SE**

(71) Applicant: **The University of Calgary
2500 University Drive N.W.
Calgary Alberta T2N 4N1 (CA)**

(72) Inventor: **Anderson, Robert
5717 Brenner Crescent, N.W.
Calgary Alberta, T2L 1Z3 (CA)**

(74) Representative: **Armitage, Ian Michael et al
MEWBURN ELLIS & CO. 2/3 Cursitor Street
London EC4A 1BQ (GB)**

(54) **Lipid based radiation protection.**

(57) A method for preventing radiation-induced damage to a surface, which comprises applying a lipid extract from a Deinococcus species to the surface in an amount sufficient to reduce damage. Lipids having an rf of at least 0.5 when measured by silica gel H thin layer chromatography in a 65:35:5 chloroform: methanol: 28% ammonia solvent system provided greater protection than slower moving lipids. A preferred lipid, which can be isolated from the natural source or prepared synthetically, is a 1,4-naphthoquinone having an isoprenoid substituent at position 2 and a lower alkyl substituent at position 3.

EP 0 263 684 A2

## Description

# LIPID-BASED RADIATION PROTECTION

This invention is directed to the preventing of radiation-induced damage to surfaces, including microorganism cultures and mammalian skin, and is particularly directed to preventing ultraviolet-light-induced damage by utilizing lipids obtained from Deinococcus radiodurans.

Deinococcus radiodurans, formerly known as Micrococcus radiodurans, has been known to be a highly radiation-resistant organism since its isolation in 1956 as reported by Anderson et al., Food Technology (1956) 10:575-577. It is, respectively, about thirty-three times and fifty-five times more resistant to ultraviolet and gamma radiation than the radiation-resistant B/r strain of Escherichia coli. Various publications have reported on biochemical constituents, such as a menaquinone system used in their classification (Yamada et al., J. Gen. Appl. Microbiol. (1977) 23:105-108), and on other aspects of their structure (Sleytr et al., Arch. Microbiol. (1976) 107:313).

A number of studies have been directed at determining the components present in D. radiodurans that are responsible for resistance to radiation. The initial studies by Anderson suggested that the resistance involved the metabolic products of sulfur-containing amino acids. These results were reported in Anderson et al., Bacteriol. Proc. 1959, 20 (1959) and Raj et al., Can. J. Microbiol., (1960) 6:289-298. Kilburn et al. Radiation Res. (1958) 9:207-215 investigated the possibility that pigments acted as an energy sink with negative results. Bruce, Radiation Research (1964) 22:155-164 obtained an aqueous extract of D. radiodurans that was capable of protecting other bacteria from the effects of ultraviolet light. In later work by the same principal author reported in Gulstein et al., Int. J. Radiat. Biol. (1978) 34:375-380, cells were extracted with n-butanol, which was then discarded, prior to demonstration of radioprotective substances in the aqueous extract. Lewis et al., Can. J. Microbiol. (1974) 20:455-459 reported that carotinoid pigments do not play any significant role in the radiation-resistance of D. radiophilus and D. radiodurans.

Since the reports indicated above do not account for the total radiation resistance of Deinococcus species, there remains a need to identify other radiation-resistant components of Deinococcus species.

The present invention provides a method of preventing radiation-induced damage to a surface by applying a lipid extract from a Deinococcus species to the surface. Specific fractions of the total-lipids extract have been identified that have in part superior protection against radiation damage. A number of individual lipid components have been isolated by thin layer chromatography. A preferred radioprotective compound is a 1,4-naphthoquinone with an isoprenoid substituent at position 2 and a lower alkyl, preferably methyl, substituent at position 3. Protection by lipid compositions of the invention against radiation-induced damage has been demonstrated for both microbial and mammalian cells.

Figure 1 is a schematic diagram of a silica gel H thin layer chromatography plate developed in the solvent chloroform: methanol: 28% ammonia (65:35:5) and identifies individual lipids and lipid fragments discussed in the specification.

Figure 2 is a schematic diagram of two silica gel H thin layer chromatography plates developed in two dimensions with different solvent systems, one of which shows separation of polar lipids and the other, non-polar lipids.

The present inventor has discovered that lipid extracts, as opposed to previous aqueous extracts, from Deinococcus species are capable of protecting surfaces from radiation damage. The radioprotective lipids can readily be obtained by extracting cells from a Deinococcus species, such as D. radiodurans or D. radiophilus with a typical lipid solvent, such as a mixture of chloroform and methanol. Mixtures of an alcohol with either a hydrocarbon solvent or halogenated (especially chlorinated) hydrocarbon solvent are particularly useful in that the ratio of components in the mixture can be varied to provide a series of extractions so that lipids with a wide range of polarities can be extracted. Useful hydrocarbons include $C_5$-$C_{10}$ alkanes and cycloalkanes, with cyclohexane being particularly useful. Examples of halogenated hydrocarbons include chlorinated methane and ethane derivatives, such as dichloromethane, chloroform, carbontetrachloride, dichloroethane, and trichloroethane, and fluorochlorocarbons, such as liquid freon compounds. Methanol and ethanol are preferred alcohols. Solvent mixtures which can be removed from extracted lipids by evaporation at a temperature of 80°C or less (optionally at reduced pressure) are preferred.

Typically, cells are disrupted and mixed with the lipid solvent and either water or an aqueous solution optionally containing salts to enhance later separation of the layers. After thorough equilibration, the layers are separated, and the organic layer containing lipids is retained. Extracted lipids can be separated from the solvent by a process such as drying on a rotary evaporator. A preferred procedure for extracting lipids of the invention is described in Thompson et al., Can. J. Microbiol. (1980) 26:1408-1411. The Deinococcus lipids can be utilized either as a total-lipids extract or the total-lipids extract can be fractionated by standard techniques of lipid chemistry, such as column chromatography, thin layer chromatography, solvent-solvent extraction, or a combination thereof.

Preferred lipid fractions are those containing lipids having an rf of 0.5 or greater when measured by silica gel H thin layer chromatography in a 65:35:5 chloroform: methanol: 28% ammonia solvent system.

Figure 1 shows thirteen individual lipids identified by thin layer chromatography of a total-lipids extract from D. radiodurans . The lipids were divided arbitrarily into four regions, shown in Figure 1, which contained lipids 1-5 (region 1), lipids 6-7 (region 2), lipids 8-9 (region 3), and lipids 10-13 (region 4).

Lipid regions 1 and 2 have been demonstrated experimentally to exhibit superior protection against radiation damage. All of the lipids in these regions have rf values of 0.5 or greater when measured in the indicated system. The individual rf values, corresponding to lipids 1-7 numbered in order of decreasing mobility, are 0.98, 0.94, 0.92, 0.88, 0.84, 0.70, and 0.50.

The structure of one of these lipids has been determined and is shown below:

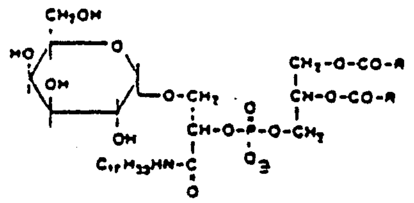

in which each R independently is the alkyl or alkenyl residue of a fatty acid, especially of 12 to 22 carbons and 0 to 3 double bonds. This is lipid 7 as shown in the Figure.

Chemical studies have identified a specific compound type (vitamin K compounds) in the lipid extract that exhibits significant radiation-protective effects. These vitamin K compounds are 1,4-naphthoquinone ring systems with an isoprenoid substituent at position 2 and a lower alkyl, typically containing 1-4 carbons, especially methyl, substituent at position 3. A general formula for this compound type is set forth below:

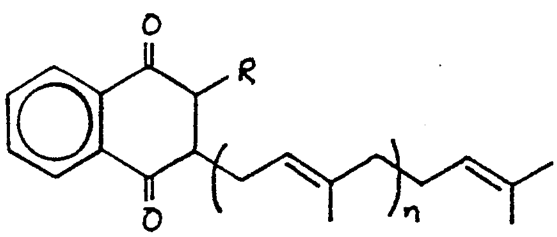

in which n is an integer from 0 to 14 preferably 3 to 10, and more preferably about 7. In extracts from D. radiodurans, fraction n2, n is equal to 7.

In the formula above, R can be any of the substituents typically found in vitamin K compounds, as are described in Sebrell and Harris, eds. The Vitamins: Chemistry, Physiology, Pathology, Methods, Ch. 10, "Vitamin K Group," 2nd ed - Vol. 3 (1971) Academic Press, New York and London, pp. 416-465. In extracts from D. radiodurans, R is methyl. Accordingly, lower alkyl groups, particularly alkyl groups containing 1-4 carbons, are preferred.

It appears that the substituents present as R, in the isoprenoid group, or else where in the molecule are not of particular importance for determining radiation-protective effects. The 1,4-napthoquinone ring system is believed to be responsible for the radiation-protective effects. Accordingly, substituents on the phenyl ring of the napthoquinone merely act to shift the wavelength of maximum protection, and can be any stable substituent normally found on an aromatic ring, such as halogen, alkyl, alkylcarbonyl, nitro, cyano, amino, alkoxy, carboxylate, and the like, in which the hydrocarbyl portion of the substituent (if present) preferably contains from 1 to 6, more preferably from 1 to 4, carbon atoms.

It should be recognized that there is no intention in this specification of indicating that all of the various molecules that can be obtained by selecting from the indicated substituents can be obtained from D. radiodurans. Rather, these are examples of equivalent compounds that can be prepared now that the property of radiation protection has been demonstrated for vitamin K compounds.

Lipids of unknown structure can be prepared using the chromatography technique set forth in this specification from natural sources, as described herein. Lipids having the specific indicated structures can readily be prepared both by isolation from natural sources and by synthetic techniques of organic chemistry. See, for example, the Sebrell and Harris text cited above for specific isolation and synthetic techniques. Typically, a synthesis will be broken down into two parts, preparation of the naphthoquinone nucleus followed by alkylation of the nucleus with a prepared isoprenoid-substituent precursor.

The specific vitamin K compound in which n = 7 and R = $CH_3$ has been shown to be the major lipid from D. radiodurans which provides radioprotective activity at 254 nm. This wavelength is generally accepted as being the one which is most active in UV-destruction of nucleic acids and living cells. While the vitamin K compounds have been demonstrated to be the major lipid having radioprotective activity at 254 nm, radioprotective activity at other wavelengths is present for other lipids and lipid fractions described herein.

A number of combinations can be made from the lipids, such as single lipids, mixtures of 2-10 (preferably 2-3) of these lipids, and total-lipids extracts. The effectiveness of any single component or mixture of components in protecting against radiation-induced damage can readily be measured by the techniques described later in this specification.

Radiation against which protection is exhibited encompasses electromagnetic radiation having wavelengths in the range from those present in ultraviolet light to those present in gamma radiation. Use of lipid extracts in protecting surfaces against ultraviolet radiation is particularly preferred.

Although lipids of the invention can be utilized in protecting any surface (such as paint, plastics, dyed surfaces, and the like) against the effects of radiation, economic considerations will likely restrict the present method to use in protecting living organisms, especially microorganisms, such as bacteria and yeast, and animal cells, especially mammalian cells, whether in cell culture or in the form of a surface, such an epidermis, of a mammal. When used to protect cell cultures, protection of hybridomas, fermentation bacteria and yeast, and the like is possible Use in sun screens intended for topical application to humans or other animals is a preferred use. All of these uses with living cells can be summarized by indicating that the surface being protected is the surface of an exogenous cell, by which is meant a cell other than the cell from which the lipid was obtained. Although the invention can be utilized to further protect Deinococcus species from radiation effects by adding exogenous lipid to a cell culture containing the organism, protection of other genera of microorganisms and of mammalian cells is preferred.

The optimum amount of lipid used in protecting a surface can readily be determined by simple experimentation utilizing either a purified lipid or a lipid extract as described herein. The lipid or lipid extract is applied to a surface at a given initial rate, preferably in the range of from 0.08 to 12 $mg/cm^2$ of surface being protected. Solutions containing at least 1.5, preferably 3.0, and more preferably 6.0, mg of lipid per ml of solution are preferred when protection by a liquid layer is possible, e.g., when protecting a cell culture medium. Sun screen lotions containing these amounts of active material are also preferred. Active ingredients can comprise from 0.2 to 98% of topical compositions, preferably 0.5 to 20% by weight.

When microbial cells are being protected, efficacy can readily be determined by measuring the number of viable cells remaining in the culture medium after exposure to ultraviolet light, as is specifically described in the following experimental section. Other methods can be utilized to determine protection of other surfaces, such as measuring color formation or loss (e.g., by reflectance spectrophotometry), increase solubility due to cross-linking (e.g., by extraction assays), and other effects normally associated with exposure to radiation.

The lipid materials can be applied in any form since protection does not appear to be tied to the vehicle used. However, it is preferred to use lipids in the form of an aqueous suspension or emulsion, particularly a cytologically or pharmaceutically acceptable aqueous carrier. The lipids may be utilized in the form of liposomes if desired. Liposomes are typically prepared by sonicating or otherwise treating a mixture of lipid and aqueous carrier, such as water or an aqueous solution containing a buffer, a salt, a dispersing agent, or the like. Liposomes may readily be produced by drying a solution of organic solvent containing the lipids, such as a chloroform solution, onto a surface and then adding the aqueous carrier to that surface. Rapid mixing or sonicating is then carried out in order to produce the lipid in the form of an aqueous emulsion, which may consist of liposomes or micelles of the lipid in the aqueous carrier matrix. When used in a cream, the lipids of the invention are generally present in higher concentration than when a micelle-containing solution or liposome-containing suspension is being produced.

Techniques for preparing solutions, dispersions, creams, ointments, and the like containing lipids are well known in the art and need not be described here in detail.

When utilized to protect microorganisms, it is preferred to prepare a suspension or solution containing the lipids present in the form of liposomes or micelles or being otherwise suspended in the growth medium in which the cells are being prepared. For example, lipids of the invention can be supplied to mammalian cells in a cell culture medium, such as Minimum Essential Medium (MEM), by supplying the liposomes suspended in MEM. Significant protection of a mouse fibroblast cell has been demonstrated utilizing a medium containing emulsified lipids of the invention at a concentration of 5 mg/ml.

The present invention also encompasses compositions containing lipids of the invention. Particu larly, the lipid compositions of the invention include total-lipids extracts from Deinococcus species as well as fractional lipid extracts containing only a portion of the total lipids. When fractional extracts are intended for later use as radioprotective substances, it is preferred to utilize a fraction containing lipids having an rf of 0.5 or greater when measured by silica gel H thin layer chromatography in a 65:35:5 chloroform: methanol: 28% ammonia solvent system. As indicated in the discussion of the method above, such fractional lipid extracts contain a number of lipids having rf values of 0.50, 0.70, 0.84, 0.88, 0.92, 0.94, and 0.98 when measured in this system. Compositions include separated components that consist essentially of a single lipid having one of these rf values, mixtures of such lipids, and total-lipids extracts.

Compositions of the invention may contain, in addition to the active lipid fractions, other lipids utilized to avoid miscability problems, oils, organic or aqueous solvents, stabilizers, pharmaceutical carriers, other radiation protective substances such as natural or artificial sun screens, and the like. Examples of carrier lipids include dioleoyl phosphatidyl choline (DOPC) and other materials that have negligible radioprotective effects but which can be utilized to avoid miscability problems with some of the less polar lipid fractions (e.g., from TLC regions 1 and 2 described above).

The invention now being generally described, the same will be better understood by reference to certain preferred examples which are included for purposes of illustration only and are not to be considered limiting of the invention unless hereafter so stated.

Example 1

Several experiments were conducted to demonstrate protection of both microbial cells and mammalian cells by lipids from Deinococcus radiodurans, either as total-lipids extracts or fractions thereof.

Materials and Methods

Lipids

Packed cells of D. radiodurans were grown on standard culture media and extracted by a modified Folsch procedure. Cells (approximately 2g wet weight) were resuspended in 10 volumes (approximately 20 ml) of $CHCl_3$:$CH_3OH$ (2:1 v/v) and gently shaken for 12 hours at room temperature. The remaining cell material was collected by filtration and then extracted with 20 ml of $CHCl_3$:$CH_3OH$ (1:2 v/v) under the same conditions. Chloroform:methanol extracts were combined and evaporated to dryness. Lipid residue was mixed with a 20-ml mixture of $CHCl_3$:$CH_3OH$:$H_2O$ (8:4:3 v/v) and let stand to allow the phases to separate. The lower phase was designated the lipid extract. The organic solvent was separated from the lower layer to give the total-lipids extract. The detailed procedure is described in Thompson et al., Can. J. Microbiol. (1980) 26:1408-1417.

The total-lipids extract was fractionated in some instances. Dried lipids resuspended in chloroform were applied to the origin of a thin layer chromatography plate (silica gel 60 or silica gel H) and developed in chloroform: methanol: 28% ammonia (65:35:5, v/v). Thirteen lipids are seen on a silica gel plate developed in this manner and are numbered from one to thirteen in order from highest to lowest mobility. A diagram of a plate developed in this manner is shown in Figure 1.

Four regions of the silica gel plate, designated regions 1-4 from highest to lowest mobility as shown in the Figure, were separated in some instances and used as fractions in the experiments described below.

The lipids were also separated into fractions by applying a chloroform solution of the total-lipids extract to a column of Bio-Sil A (bio-rad) silicic acid prepared in chloroform. Sequential elution of the lipids from this column was performed with discontinuous 2-column volumes of chloroform: methanol: 28% ammonia in the following ratios: 90:10:1, 80:20:2, 70:30:3, 60:40:4, and 50:50:5, v/v. Lipids were eluted into different fractions. For example, the 60:40:4 eluate contained a mixture of two lipids, designated lipids 6 and 7 in Figure 1.

Purified lipids for use in comparisons were obtained from the following sources: dioleoyl phosphatidyl choline (Sigma Cat. No. P-1013): dilinoleoyl phosphatidyl choline (Sigma Cat. No. P-7649); egg phosphatidyl choline (purified by preparative thin layer chromatography).

Irradiation Protocol

Irradiations were conducted in wells of a 24-well plastic microtiter plate (Nunc Plastics). A General Electric G30T8 30-watt germicidal lamp was used to irradiate the wells of the microtiter plate from above through air at a distance of 10-12, or 20 cm (E. coli studies) or 30 cm (fibroblast studies) at room temperature for the times indicated. Samples were removed at the indicated intervals, usually ten seconds apart, and the viability of the aliquots were determined.

Viability Assay

Aliquots from the irradiation protocol were analyzed for viability utilizing different assays for E. coli and fibroblast cells.

With E. coli cells, aliquots (generally 10 μl) were plated onto nutrient agar and incubated overnight at 37° C. Viability was determined by colony counting.

With fibroblast cells, viability was measured by incorporation of radioactive thymidine. After irradiation, aliquots from individual wells were supplemented with one μCi of $^3H$-thymidine and incubated 30 minutes at 37° C. The incorporation of radioactive thymidine into cellular DNA was assayed using standard precipitation with trichloroacetic acid. Measurement of radioactivity was by scintilation spectrometry.

Fibroblast Cell Culture

Mouse L-2 fibroblasts described in Rothfels et al., Canadian Cancer Conf. (1959) 3:189-214, were cultured in Eagle's Minimum Essential Medium (MEM) supplemented with 5% fetal calf serum (FCS). For the irradiation protocol described above, cultures were established in wells of a 24-well microtiter plate. Upon reaching confluency (2-4 × $10^5$ cells/well), the medium was changed to MEM (without FCS) just prior to lipid supplementation and irradiation. A volume of 0.5 ml (MEM) was employed per well.

Lipid Preparation and Supplementation for Fibroblast Cells

Either dioleoyl phosphatidyl choline or a lipid extract from D. radiodurans prepared as described above was emulsified in MEM (2-4 minutes mixing with Fisher Scientific "Vortex-Genie" Cat. No. 12-812) at a concentration of 5 mg/ml. For the addition of lipid to the mouse L-2 cells, medium was removed from the wells of the microtiter plate and replace with 0.5 ml of MEM containing the emulsified lipid or lipids.

Lipid Preparation and Supplementation to E. coli

Lipids were prepared as described above. E. coli cells, grown on 0.5% tryptone (Difco Cat. No. 0123-01) and 0.3% yeast extract (Difco Cat. No. 0127-01), were washed with water and placed into wells at a cell count between 3 × $10^6$ and 1 × $10^7$ in 0.25 ml water. The indicated amount of lipid was then added to each well.

Results

In a preliminary trial to evaluate the ability of membrane lipids from D. radiodurans to afford radio-protection toward an ultraviolet-sensitive target, a total lipid extract was prepared from D. radiodurans, sonicated in water to produce a liposome suspension, and the resultant liposomes mixed with cells from E. coli (7 × $10^7$ cells in 300 μl water). The mixtures were exposed for 0, 10, 20, or 30 seconds to an ultraviolet lamp at a flux of approximately 6.3 $W/m^2$ positioned 12 cm above the samples. Following irradiation, numbers of viable E. coli were assayed by colony counts. The results, even at the lowest concentration tested (400 μg/ml), indicated a very strong radioprotective effect. Both 800 and 400 μg/ml liposome preparations exhibited high survival rates (greater than 70%) even after 30 seconds irradiation. In contrast, a logarithmic decrease in viability was seen with E. coli cells not containing the protective liposomes prepared from D. radiodurans lipids. Survival after 30 seconds irradiation was less than 2% for the control sample.

Further experiments were conducted to determine whether protection of E. coli was generally given by lipids or was specific to lipids from D. radiodurans.

In the first of these experiments, different concentrations of a total-lipids extract from D. radiodurans were utilized with an E. coli cell preparation. As expected, protection varied with the concentration of the lipids utilized. For example, after irradiation for 80 seconds at a distance of 20 cm as described above, over 80% of E. coli cells (originally 3 × $10^6$ cells and 0.25 ml water) survived when a lipid extract from D. radiodurans was present at a concentration of 13.6 mg/ml. Approximately 20% survived when protected at a concentration of 5.4 mg/ml and approximately 0.2% survived at a concentration of 2.7 mg/ml. In contrast, a well containing no lipid had a survival rate of approximately 0.003% under the same conditions.

In contrast, no statistically significant enhanced survival rate of cells was seen for dioleoyl phosphatidyl choline or dilinoleoyl phosphatidyl choline at a concentration of 10 mg/ml. Egg phosphatidyl choline did show some protection but less than that for lipids extracted from D. radiodurans. Egg phosphatidyl choline at a concentration of 10 mg/ml resulted in a survival rate of less than 1% after 80 seconds irradiation and less than 0.01% after an equal amount of irradiation for a solution containing 5 mg/ml. In contrast, as discussed above, there was a greater than 20% survival rate for cells irradiated in the presence of a liquid extract from D. radiodurans at a concentration of 5.4 mg/ml, an enhanced survival rate of two thousandfold for these conditions of irradiation.

Similar results were seen in a study measuring the protection of mouse L-2 fibroblast cells utilizing either a fractional lipid extract from D. radiodurans or dioleoyl phosphatidyl choline. Greater than 10% survival was seen for D. radiodurans-protected cells while cell cultures containing dioleoyl phosphatidyl choline were not significantly different from control samples not containing any added lipid.

The experiments set forth above utilized total-lipids extracts. In order to determine variability in protection between the various components, lipids were divided into four regions on a thin layer chromatography plate as shown in the Figure. In order to avoid miscability problems with some of the less polar lipid fractions (i.e., lipids from TLC regions 1 and 2), liposomes were prepared using carrier dioleoyl phosphatidyl choline, which the experiments above had shown to exhibit negligible radioprotective effects. Aliquots of fractionated lipids from D. radiodurans (fractions 1-4 by thin layer chromatography) were mixed in a 1:1 weight ratio with dioleoyl phosphatidyl choline and emulsified by mechanical vortexing for 2-4 minutes at room temperature in water. The final concentration of lipid was 1.8 mg/ml (excluding dioleoyl phosphatidyl choline).

The lipid preparations were added to freshly harvested cells of E. coli (1 × $10^7$ cells in a 200 μl final volume). The wells were irradiated at room temperature from a distance of 25 cm for 0-80 seconds. Aliquots were removed at intervals and assayed for cell viability by colony count formation in nutrient agar.

It should be noted that in this study, lipid concentrations used (1.8 mg/ml) were lower than those described for the initial studies above (2.7-13.6 mg/ml). Hence, less radioprotection was expected, and less radioprotection was observed. Nevertheless, it was apparent that, on an equal weight basis, fractions 1 and 2 (from thin layer chromatography regions 1 and 2) were more effective radioprotectants than fractions 3 and 4. However, all four fractions did offer some protection compared to the control sample not containing any lipid. After 20 seconds irradiation, fractions 1 and 2 protected E. coli approximately 40% better than fractions 3 and 4. After 60 seconds irradiation, protection differed for fractions 1 and 2 versus fractions 3 and 4 by greater than ten-fold.

Example 2

Several additional experiments were conducted using a different chromatography system to develop and isolate individual lipids.

Material and Methods

Lipids

Total lipids, extracted from D. radiodurans according to the procedure of Thompson et al., Can. J. Microbiol. (1980) 26:1408-1411, were separated using two-dimensional thin-layer chromatography (TLC) in two solvent systems in order to distinguish polar lipids (System A) from nonpolar lipids (System B). System A utilized the solvents chloroform/methanol/28% ammonia (65:35:5) in the first dimension and chloroform/acetone/methanol/acetic acid/water (10:4:2:2:1) in the second dimension. System B utilized the solvents hexanes/ether/acetic acid (50:50:1: half development) and hexanes/ether/acetic acid (90:10:1; full development) in the first dimension and chloroform/ether (90:10) in the second dimension. For convenience in detecting lipids on chromatograms, D. radiodurans was cultured in the presence of $^{14}C$-acetate. Radiolabeled lipids were then detected by autoradiographic TLC (Figure 2).

In the left-hand portion of Figure 2, 13 individual lipids can be seen along with a blot in the upper-left-hand portion of the diagram showing the polar lipids which represents all of the non-polar lipids running together. These non-polar lipids are spread out over the chromatograph diagram shown in the right-hand portion of Figure 2 using solvent system B. The polar lipids can be seen as a single blotch in the lower-right-hand portion of the non-polar diagram.

For the preparative isolation of selected lipids or groups of lipids, column chromatography was employed. Total lipids, extracted from D. radiodurans, and dissolved in hexanes/acetic acid (99:1) were applied to a column of Biosil A (BioRad) and eluted sequentially with an increasing gradient of chloroform in hexanes/acetic acid (99:1) and finally with an increasing gradient of methanol/28% ammonia (10:1) in chloroform. Fractions of 9 ml were collected from the column and pooled to yield lipid groups that were tested for radioprotective activity as described below.

Radioprotection of E. coli by Isolated Lipids from D. radiodurans

Column chromatographic lipid pools were incorporated into dioleoyl phosphatidyl choline (DOPC) liposomes and assayed for their ability to protect E. coli from killing by UV light at 254 nm. Respective lipids or lipid mixtures were incorporated into DOPC liposomes (4 mg/ml) at concentrations of 1 mg/ml in water. Freshly grown and water-washed suspensions of E. coli ($10^8$ colony forming units/ml) were irradiated under UV for various times and their survival times quantitated by colony counts. $D_{37}$ values were determined from plots of log survival versus dose. $D_{37}$ is the dose of ultraviolet light in $J/m^2$ required to reduce the number of viable cells to 37% of the original number. As can be seen from Table 1 below, the highest radioprotective activity was found associated with the lipid designated n2.

Table 1

Radioprotection of E. coli by Isolated Lipids from D. radiodurans

| Lipid Designation | $D_{37}$ ± SD ($J/m^2$) | (n) |
|---|---|---|
| DIPC liposomes alone | 67 ± 20 | (3) |
| + n1 | 78 ± 29 | (2) |
| + n2 (vitamin K) | 310 ± 17 | (2) |
| + n3-n8 | 77 ± 17 | (2) |
| + 1-5 | 105 ± 35 | (2) |
| + 6-7 | 98 ± 29 | (6) |
| + 8-9 | 78 ± 29 | (3) |
| + 10-13 | 75 ± 7 | (2) |

Demonstration the Lipid n2 is a Vitamin K

Nuclear magnetic spectroscopy (NMR) of isolated lipid n2 demonstrated the presence of a naphthoquinone ring structure, thus defining a vitamin K class of compounds. Ultraviolet spectroscopy also confirmed the naphthoquinone ring structure. Lipid n2 co-chromatographed with authentic vitamin $K_1$ (Sigma) on TLC in two solvent systems: hexanes/ether/acetic acid (90:10:1) and chloroform/ether (90:10). A vitamin K (MK-8) has previously been identified in D. radiodurans (Yamada et al., op. cit.) as a marker for use in identifying the species (but apparently not as a source of radioprotective activity).

Radioprotection by Vitamin K of Skin of Hairless Mice

Eight-month-old female hairless mice (albino Skh:hairless-1) were irradiated on their back surfaces with ultraviolet light at a distance of 20 cm for 2 minutes (UV intensity, 5 $W/m^2$). Prior to irradiation, the mice were treated with 35 µl (spread over 15 $cm^2$) aqueous DOPC mixtures (10 mg/ml) containing vitamin K at concentrations of 0, 0.2, 0.5, 1, or 2 mg/ml. Following irradiation, the mice were returned to their normal cages and examined for evidence of UV skin damage (erythema and edema). A five-plus scaling system was used to evaluate degree of damage. Examinations were performed blindly (i.e., without knowledge of treatment protocol by the examiner) and independently by a total of eight researchers, and the cumulative results were averaged. As can be seen from Table 2 below, there is a clear correlation between UV-induced skin damage and decreasing amount of vitamin K.

Table 2

Radioprotection of UV-irradiated Mouse Skin by Vitamin K

| Treatment | Severity of UV-induced Damage* (edema and erythema) |
|---|---|
| DOPC alone | 4.5 ± 0.7 |
| DOPC + 0.2 mg/ml vitamin K | 3.9 ± 0.8 |
| DOPC + 0.5 mg/ml vitamin K | 3.0 ± 0.9 |
| DOPC + 1.0 mg/ml vitamin K | 2.0 ± 1.1 |
| DOPC + 2.0 mg/ml vitamin K | 1.5 ± 0.6 |

* Assessed 24 hrs after irradiation using a 1-5 severity scale. Results are expressed as average ± standard deviation of the assessments of eight independent, uninitiated observers.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

The invention now being fully described, it will be apparent to one of ordinary skill in the art that many changes and modifications can be made thereto without departing from the spirit or scope of the appended claims.

**Claims**

1. A method of protecting a surface against electromagnetic radiation induced damage, which comprises applying to said surface a lipid extract from a Deinococcus species.
2. A method according to claim 1 wherein said surface is a surface of an exogenous cell.
3. A method according to claim 2, wherein said cell is a bacterium or yeast.
4. A method according to claim 2, wherein said cell is a mammalian epidermal cell.
5. A method according to any one of the preceding claims wherein said extract is applied in an amount from 0.08 to 12 $mg/cm^2$.
6. A substance capable of protecting a surface against electromagnetic radiation induced damage, said substance comprising a lipid extract from a Deinococcus species, said extract containing other than merely vitamin K compounds.
7. A substance according to claim 6 for use in the protection of a surface against electromagnetic radiation induced damage.
8. A topical composition for the treatment of the human or animal epidermis to inhibit electromagnetic radiation induced damage which composition comprises a lipid extract from a Deinococcus species.
9. The use of a lipid extract from a Deinococcus species in the manufacture of a preparation for

protecting a surface against electromagnetic radiation induced damage.

10. A method, substance, composition or use according to any one of the preceding claims, wherein said species is Deinococcus radiodurans.

11. A method, substance, composition or use according to any one of claims 1 to 9 wherein said species is Deinococcus radiophilus.

12. A method substance, composition or use according to any one of the preceding claims wherein said lipid extract is a total lipid extract.

13. A method, substance, composition or use according to any one of claims 1 to 11, wherein said lipid extract is a fractional lipid extract.

14. A method, substance, composition or use according to claim 13, wherein lipids present in said fractional lipid extract have an rf of 0.5 or greater when measured by silica gel H thin layer chromatography in a 65:35:5 chloroform: methanol: 28% ammonia solvent system.

15. A method, substance, composition or use according to claim 14, wherein said fractional lipid extract contains a lipid having an rf value of 0.50, 0.70, 0.84, 0.88, 0.92, 0.94 or 0.98 when measured in said system.

16. A method, substance, composition or use according to claim 15, wherein said extract consists essentially of a single lipid of said rf value.

17. A method, substance, composition or use according to claim 15 or claim 16. wherein said lipid is a 1,4-naphthoquinone having an isoprenoid substituent at position 2 and a lower alkyl substituent at position 3.

18. A method, substance, composition or use according to any one of the preceding claims, wherein said lipid extract is prepared in the form of an aqueous emulsion.

19. A method of protecting a surface against electromagnetic radiation induced damage, which comprises applying to said surface a compound of formula

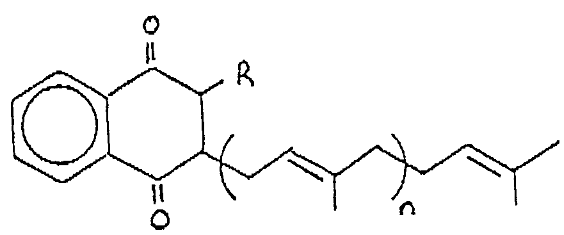

wherein:

n is an integer from 0 to 14 and R is a $C_1$-$C_4$ alkyl group,

20. Use of a compound of formula

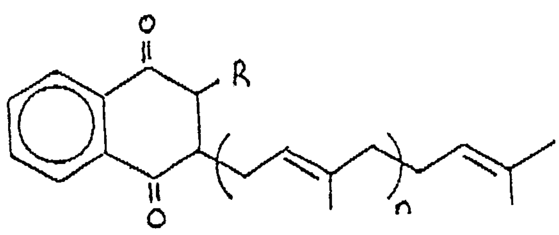

wherein:

n is an integer from 0 to 14 and R is a $C_1$-$C_4$ alkyl group;

in the manufacture of a preparation for protecting a surface against electromagnetic radiation induced damage.

21. A topical composition for the treatment of the human or animal epidermis to inhibit electromagnetic radiation induced damage, which composition comprises a compound of formula

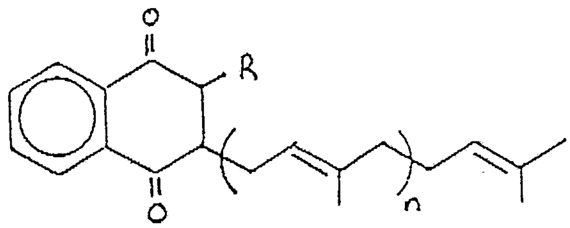

wherein :

n is an integer from 0 to 14

and R is a $C^1$- $C_4$ alkyl group;

22. A method, use or composition of any one of claims 19, 20 or 21 wherein n is 7 and R is methyl.

23. A method, substance, composition or use according to any one of the preceding claims wherein said radiation is ultraviolet radiation.

Claims for the following Contracting States: AT; ES; GR

1. A method of protecting a surface against electromagnetic radiation induced damage, which comprises applying to said surface a lipid extract from a Deinococcus species.

2. A method according to claim 1 wherein said surface is a surface of an exogenous cell.

3. A method according to claim 2, wherein said cell is a bacterium or yeast.

4. A method according to claim 2, wherein said cell is a mammalian epidermal cell.

5. A method according to any one of the preceding claims wherein said extract is applied in an amount from 0.08 to 12 mg/$cm^2$.

6. A substance capable of protecting a surface against electromagnetic radiation induced damage, said substance comprising a lipid extract from a Deinococcus species, said extract containing other than merely vitamin K compounds.

7. A substance according to claim 6 for use in the protection of a surface against electromagnetic radiation induced damage.

8. A process which comprises the preparation of a topical composition for the treatment of the human or animal epidermis to inhibit electromagnetic radiation induced damage which composition comprises a lipid extract from a Deinococcus species.

9. The use of a lipid extract from a Deinococcus species in the manufacture of a preparation for protecting a surface against electromagnetic radiation induced damage.

10. A method, substance, process or use according to any one of the preceding claims, wherein said species is Deinococcus radiodurans.

11. A method, substance, process or use according to any one of claims 1 to 9 wherein said species is Deinococcus radiophilus.

12. A method substance, process or use according to any one of the preceding claims wherein said lipid extract is a total lipid extract.

13. A method, substance, process or use according to any one of claims 1 to 11, wherein said lipid extract is a fractional lipid extract.

14. A method, substance, process or use according to claim 13, wherein lipids present in said fractional lipid extract have an rf of 0.5 or greater when measured by silica gel H thin layer chromatography in a 65:35:5 chloroform: methanol: 28% ammonia solvent system.

15. A method, substance, process or use according to claim 14, wherein said fractional lipid extract contains a lipid having an rf value of 0.50, 0.70, 0.84, 0.88, 0.92, 0.94 or 0.98 when measured in said system.

16. A method, substance, process or use according to claim 15, wherein said extract consists essentially of a single lipid of said rf value.

17. A method, substance, process or use according to claim 15 or claim 16, wherein said lipid is a 1,4-naphthoquinone having an isoprenoid substituent at position 2 and a lower alkyl substituent at position 3.

18. A method, substance, process or use according to any one of the preceding claims, wherein said lipid extract is prepared in the form of an aqueous emulsion.

19. A method of protecting a surface against electromagnetic radiation induced damage, which comprises applying to said surface a compound of formula

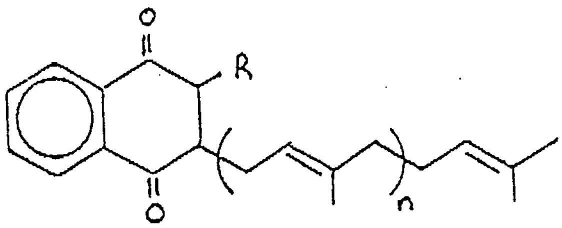

wherein:

n is an integer from 0 to 14
and R is a $C_1$-$C_4$ alkyl group;

20. Use of a compound of formula

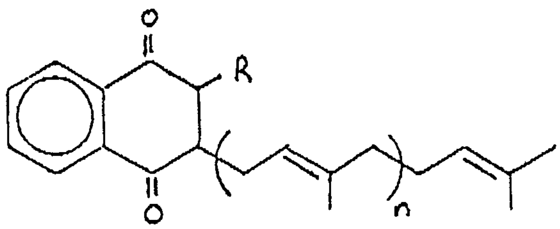

wherein:

n is an integer from 0 to 14
and R is a $C_1$-$C_4$ alkyl group;

in the manufacture of a preparation for protecting a surface against electromagnetic radiation induced damage.

21. A method, or use of claim 19 or claim 20 wherein n is 7 and R is methyl.

22. A method, substance, process or use according to any one of the preceding claims wherein said radiation is ultraviolet radiation.

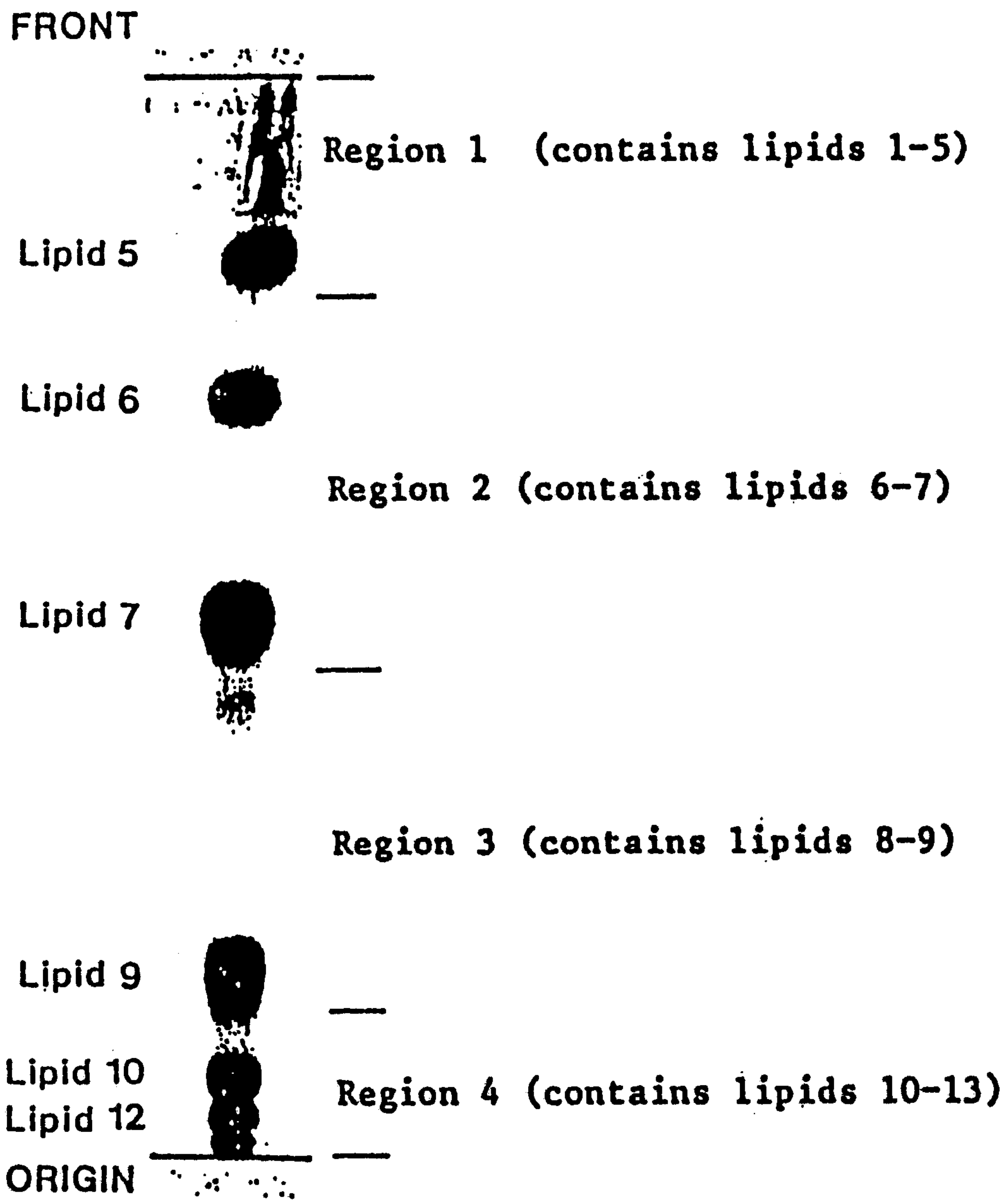
FRONT
Region 1 (contains lipids 1-5)
Lipid 5
Lipid 6
Region 2 (contains lipids 6-7)
Lipid 7
Region 3 (contains lipids 8-9)
Lipid 9
Lipid 10
Lipid 12
Region 4 (contains lipids 10-13)
ORIGIN

FIG. 1

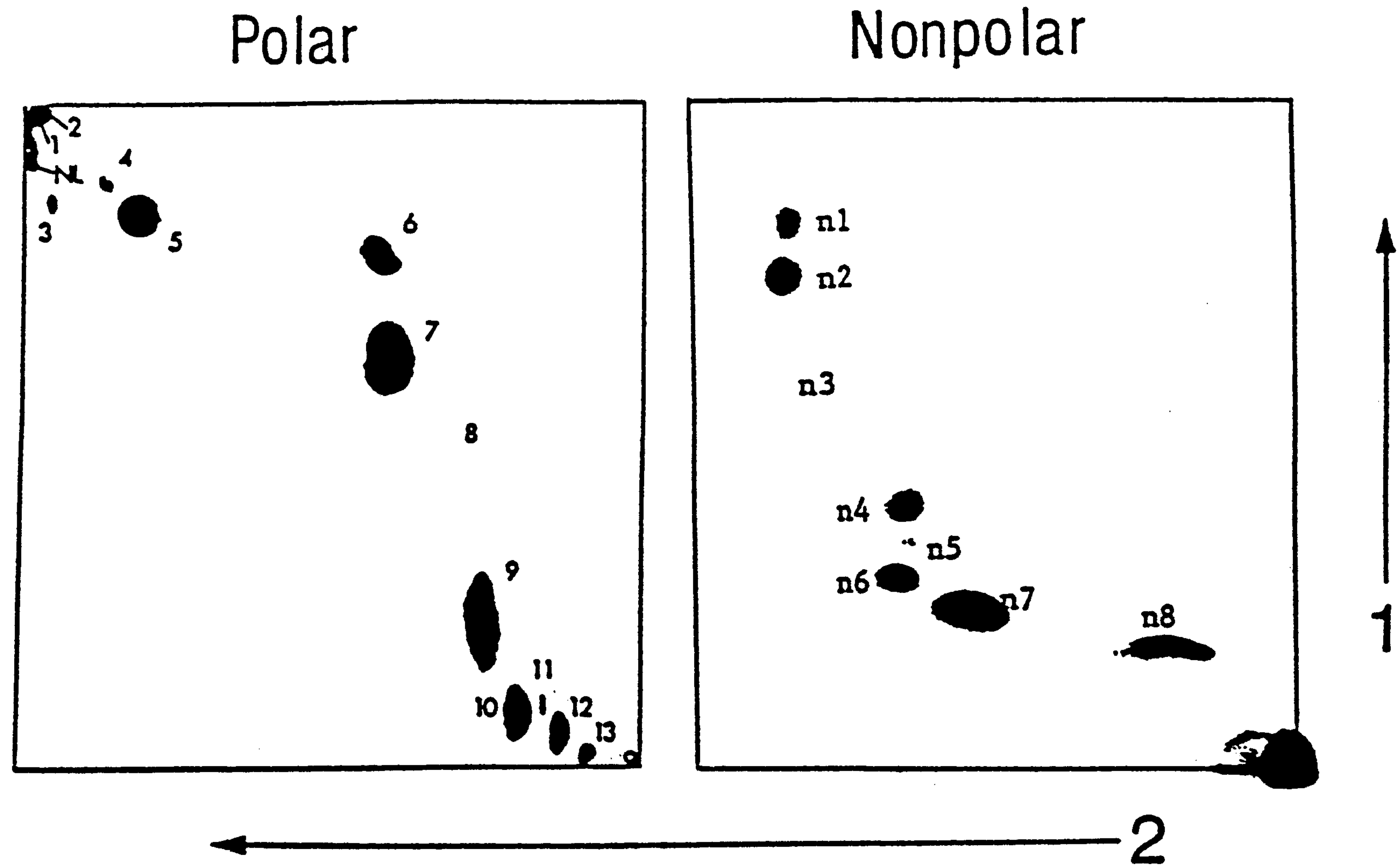


FIG. 2